# EUROPEAN PATENT SPECIFICATION

(11) **EP 0 664 108 B1**
(45) Date of publication and mention of the grant of the patent: **21.08.2002**
(21) Application number: 95200164.2
(22) Date of filing: 24.01.1995
(51) Int. Cl.: A61F 2/40, A61F 2/30

(54) **Shoulder endoprosthesis**
Schultergelenk-Endoprothese
Endoprothèse d'épaule

(30) Priority: 24.01.1994 DE 4401952
(43) Date of publication of application: 26.07.1995
(73) Proprietor: ESKA Implants GmbH & Co., 23556 Lübeck (DE)
(72) Inventor: Schug, Martin, D-2355 2 Lübeck (DE); Rozing, Perrus M., Prof. Dr., NL-2361 SH Warmond (NL)
(74) Representative: Weiss, Christian, Dipl.-Ing.

(56) References cited:
- EP-A- 0 329 854
- EP-A- 0 342 421
- EP-A- 0 457 222
- EP-A- 0 549 480
- DE-A- 4 220 217
- FR-A- 2 579 454
- FR-A- 2 631 544
- FR-A- 2 664 809
- US-A- 3 869 730
- US-A- 4 919 670
- US-A- 4 986 833

## Description

The invention relates to a shoulder endoprosthesis with a humeral portion, comprising a stalk that can be anchored in the humerus, a collar at the proximal end of the stalk, and a joint head on the collar in the form of a segment of a sphere on the collar, wherein the collar is designed as an essentially circular disk and wherein the joint head extends radially from its axis of rotation at least to the periphery of the collar.

Such a shoulder endoprosthesis is known, for example, from European Patent Application 0 329 854 or FR-A-2664809. In this known prosthesis a collar, part of which projects radially beyond the collar and which provides positioning on the resected edge of the bone is placed at the proximal end of the stalk.

However, the long-term anchoring of the shoulder endoprosthesis in the humerus is not always satisfactory, especially when no cement is used.

The object of this invention is, therefore, to mitigate the disadvantages of the prior art so as to assure reliable fixation of the prosthesis and reliable functioning of the prosthesis.

This object is attained according to the invention by a shoulder endoprosthesis of the type initially specified, such that the collar has on the side towards the bones, at least in sections, an open-cell or open-pore surface structure , in that at least two wings branch radially from the stalk, which extend to the underside of the collar and in that the wings have, at least in sections, an open-cell or open-pore surface structure.

Accordingly, it is provided that the spongy bone can grow into it because of its open-cell or open-pore surface structure on the lower side. That substantially improves the long-term fixation of the humeral part in the humerus, correspondingly reducing the danger of a dislocation. Further, the shoulder endoprosthesis contains at least two wings, which branch off radially from the stalk, separated by about 90°, and which end at the lower side of the collar. It is desirable for the wings to be directed medially. An embodiment with four wings is also conceivable, with two of the wings directed dorsally and two medially. It is essential for the wings to have, at least in sections, an open-cell or open-pore surface structure. The wings can accept forces acting transversely to the humerus on the joint head, and improve the total fastening of the humeral part in the humerus. Growth of spongy bone material into the open-cell or open-pore surface structure of the wings gives a particularly intimate joint between the bone and the prosthesis.

It is particularly desirable for the joint head to extend radially from its axis of rotation to the periphery of the collar, axially immediately adjoining the periphery of the collar. Thus the periphery of the collar, properly shaped, can form a spherical cavity along with the joint head, all of which is available as a joint surface. In this manner, the limited space available for the joint head is utilized optimally. The effective joint surface extends to the resected edge of the humerus.

As an alternative to this, in another especially preferred embodiment of the invention, the joint head also extends axially beyond the periphery of the collar. In this embodiment, also, no significant spaces are produced between the resected edge and the joint head. The joint head extends past the collar axially and radially, and the edge of this section lies immediately adjacent to the resected edge of the humerus. This embodiment utilizes the space available in the shoulder region for the joint head especially well. The large joint area assures reliable functioning of the prosthesis.

It is particularly desirable for the joint head to be mounted removably on the collar, so that a suitable joint head for the patient can be selected during surgery, if there is a suitable supply of different sizes of joint heads. Tapered plug joints between the joint head and collar have proved advantageous. Tapered plug joints are self-limiting, so that they are particularly simple connections.

It has also proved advantageous to have the conical cavity on the underside of the joint head with the tapered plug on the collar, so as to keep the height of the humerus part as low as possible.

It is particularly preferable to place a coupling element between the joint head and the collar. The coupling element has an inner cone which can be placed on the tapered plug of the collar and an outer cone which can be placed in the conical cavity on the underside of the joint head. The principal advantages of this particularly preferred embodiment are that, given a proper supply during the surgery, it is possible to choose not only the size of the joint head but also the distance between the plateau and the joint head. It is preferable for the axes of rotation of the inner cone and the outer cone of the coupling element to be at a specified angle with each other. In this way, the antetorsional or retrotorsional angle of the joint head can be selected, and adjusted during the surgery, to meet the requirements of the patient.

It is particularly preferred to have a hole through the collar and the proximal segment of the stalk, through which a bone screw can be placed to anchor the humerus part in the humerus. In this case it is desirable for the axes of rotation of the joint head and the hole to coincide. That makes it possible for the surgeon to introduce a bone screw into the hole in the collar, projecting laterally to the stalk, which can be anchored in the spongy tissue of the humerus to achieve sufficient primary fixation of the humeral part in the humerus.

It is advantageous for the stalk to have an essentially circular cross section tapering toward its distal end so as to match the anatomic details of the humerus.

In the following, embodiments of the invention are described by means of the drawings. They show:
- Figure 1.: a cross-section of a humeral part of a shoulder endoprosthesis anchored in the humerus;
- Figure 2.: a cross-section through another embodiment of the humeral part;
- Figure 3.: a cross-section through the stalk and the wing of the humeral part;
- Figure 4.: a cross-section through a coupling element;

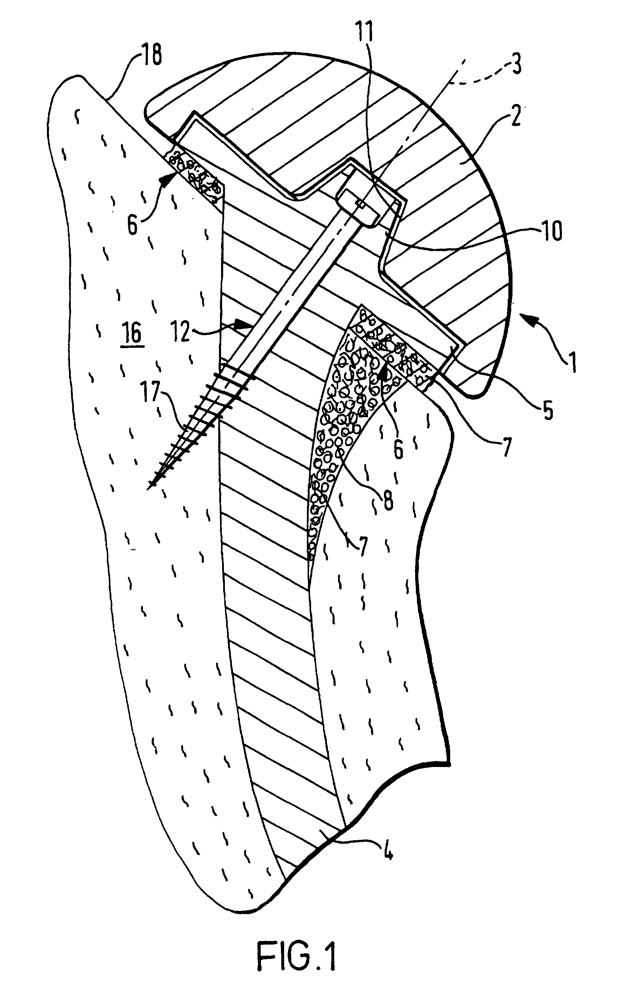

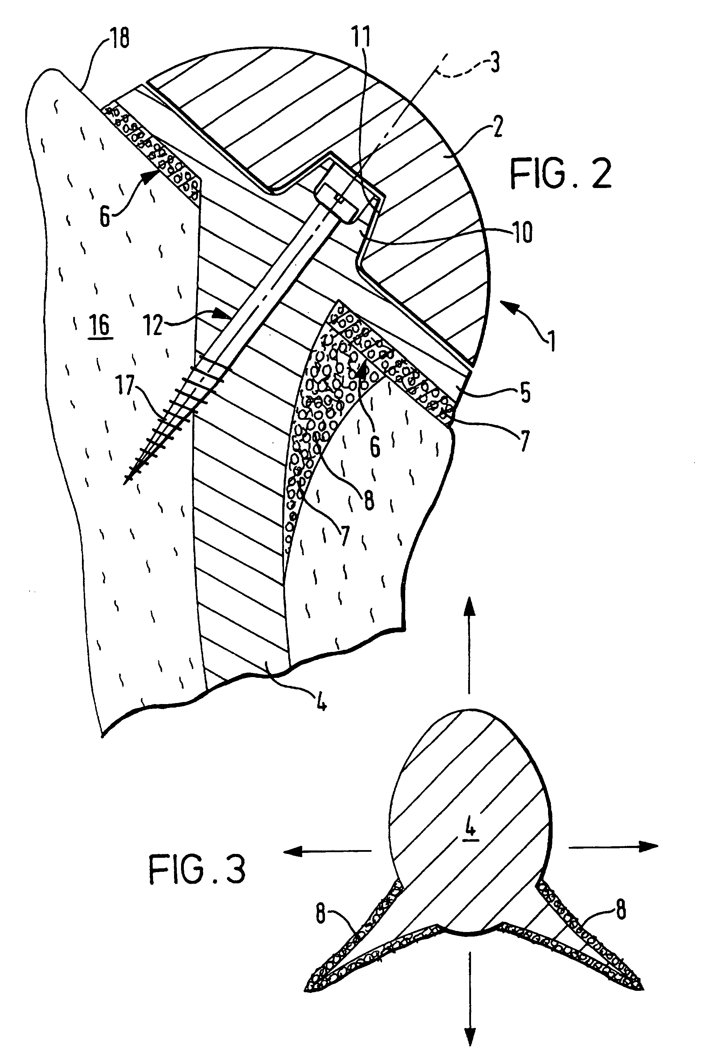

Figures 1 and 2 show a humeral part **1** of a shoulder endoprosthesis having a joint head **2** in the shape of a segment of a sphere, which is anchored in the humerus **16** with a stalk **4**.

Two wings 8, 90° apart, branch off from the stalk **4** in the medial direction, tapering toward the outside. The wings **8** and the stalk **4** are also shown in cross-section in Figure 3. The wings 8 provide better transfer of transverse forces and have an open-cell or open-pore surface structure **7** into which the bone material can grow for better fixation to the bone. The radial extent of the wings **8** increases from distal to proximal, corresponding to the anatomic details of the human humerus **16**.

A collar **5**, designed as an essentially circular disk, is placed at the proximal end of the stalk **4**. The collar **5** lies with its distal underside **6** on the resected edge **18** of the humerus **16**. An open-cell or open-pore surface structure **7**, into which the spongy tissue can grow for better fixation of the humeral part **1** to the humerus **16**, is provided on the underside **6** of the collar **5**. There is a tapered plug **10** on the collar **5**, onto which the joint head **2** can be plugged with the corresponding conical cavity **11** on its distal underside. A hole **12** passes through the tapered plug **10**, the collar **5** and the stalk **4** to accept a bone screw **17**. The rotational axis of the hole coincides essentially with that of the tapered plug **10**. The through-hole **12** can be widened in the tapered plug **10** to accept the head of the bone screw **17**. The bone screw **17** is introduced into the through-hole **12** from above during the surgery, before the joint head **2** is set in place. It emerges from the lateral side of the stalk **4** and is screwed into the spongy tissue of the humerus **16**.

The joint head **2**, formed as a segment of a sphere, has a cavity corresponding to the collar **5** at its underside and projects both radially and axially beyond the collar **5** in the embodiment shown in Figure 1. Thus the surface of the joint head **2** extends nearly to the resected edge **18** of the humerus **16**.

In spite of the large joint area, the overall height of the humeral part **1** above the resected edge **18** is small, so that the physiological center of the joint is maintained.

The same advantages can also be attained with the embodiment shown in Figure 2, in which the joint head **2** extends radially from its axis of rotation to the periphery of the collar **5** and immediately adjoins the periphery of the collar **5**. The shape of the periphery of the collar **5** is matched to the surface of the joint head **2** and, together with the joint head, forms a spherical vault, all of which is available as joint surface.

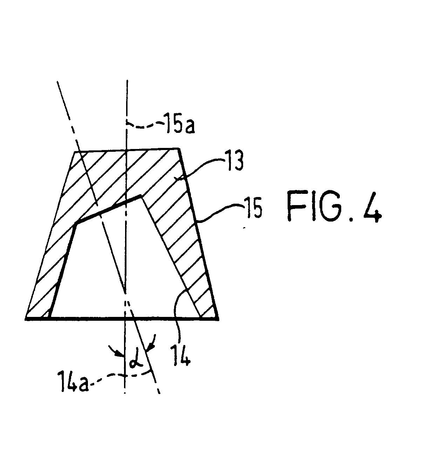

By means of the tapered plug joint between the plateau **5** and the joint head **2** it is possible, during the surgery and after the stalk **4** has been mounted in the resected humerus **16**, to select a joint head **2** which appears to be of suitable size. Furthermore, a separate coupling element **13** can be placed between the joint head **2** and the plateau **5**, as shown in cross section in Figure 4. The coupling element **13** has an inner cone **14** and an outer cone **15**, fitting the tapered plug **10** and the conical cavity **11**, respectively. The axes of rotation **14a** and **15a** of the inner cone **14** and the outer cone **15**, respectively of the coupling element make a specified angle α with each other. If necessary the angle α can be 0°. By choice of a suitable coupling element it is possible to adjust not only the antetorsional and retrotorsional angles but also the distance between the plateau **5** and the joint head **2**.

## Claims

1. Shoulder endoprosthesis having a humeral part (1) comprising a stalk (4) that can be anchored in the humerus, a collar (5) at the proximal end of the stalk (4), and a joint head (2) formed as a segment of a sphere on the collar (5), wherein the collar (5) is designed as an essentially circular disk and wherein the joint head (2) extends radially from its axis of rotation (3) at least to the periphery of the collar (5),
**characterized in that**
the collar (5) has on its side (6) Facing towards the bones, at least in sections, an open-cell or open-pore surface structure (7), **in that** at least two wings (8) branch radially from the stalk (4), which extend to the side (6) of the collar facing towards the bone (5) and **in that** the wings (8) have, at least in sections, an open-cell or open-pore surface structure (7).

2. Shoulder endoprosthesis according to one of the claims 1, **characterized by** the fact that at least two wings (8) are directed medially.

3. Shoulder endoprosthesis according to claim 1, **characterized by** the fact that the joint head (2) extends radially from its axis of rotation (3) to the periphery of the collar (5).

4. Shoulder endoprosthesis according to claim 3, **characterized by** the fact that the periphery of the collar (5) is axially adjacent the joint head (2).

5. Shoulder endoprosthesis according to claim 4, **characterized by** the fact that the periphery of the collar (5) is matched to the surface of the joint head (2) so that the joint head (2) and the collar (5) form a spherical vault.

6. Shoulder endoprosthesis according to claim 1, **characterized by** the fact that the joint head (2) projects axially beyond the periphery of the collar (5).

7. Shoulder endoprosthesis according to one of the preceding claims, **characterized by** the fact that the joint head (2) is removably fastened to the collar (5).

8. Shoulder endoprosthesis according to one of the preceding claims, **characterized by** a tapered-plug connection (10, 11) between the joint head and the collar (5).

9. Shoulder endoprosthesis according to claim 8, **characterized by** the fact that there is a conical cavity (11) on the underside of the joint head (2) and a tapered plug (10) on the collar (5).

10. Shoulder endoprosthesis according to one of the preceding claims, **characterized by** a coupling element (13) between the joint head (2) and the collar (5), having an inner cone (14) and an outer cone (15) so that the inner cone (14) can be coupled with the tapered plug (10) and the outer cone (15) can be coupled with the conical cavity (11).

11. Shoulder endoprosthesis according to one of the preceding claims,
**characterized by** the fact the rotational axis (3) of the joint head (2) is at a specified angle to the side (6) of the collar (5) facing towards the bone.

12. Shoulder endoprosthesis according to claim 10 or 11 **characterized by** the fact that the rotational axes of the inner cone (14) and the outer cone (15) of the coupling element (13) make a specified angle α with each other.

13. Shoulder endoprosthesis according to one of the preceding claims, **characterized by** the fact that there is on the underside of the joint head (2) a cavity matching the collar (5).

14. Shoulder endoprosthesis according to one of the preceding claims, **characterized by** the fact that the stalk (4) tapers toward its distal end.

## Patentansprüche

1. Schulter-Endoprothese mit einem humeralen Teil (1), der einen Stiel (4) umfasst, welcher im Humerus verankert werden kann, einen Stiel (5) am proximalen Ende des Stiels (4) und einen Gelenkkopf (2), geformt als Abschnitt einer Kugel auf dem Kragen (5), wobei der Kragen (5) als eine im Wesentlichen kreisförmige Scheibe konzipiert ist und wobei sich der Gelenkkopf (2) von seiner Rotationsachse (3) aus radial bis mindestens zum äußeren Bereich des Kragens (5) erstreckt,
**gekennzeichnet dadurch, dass**
der Kragen (5) auf seiner Seite (6), die den Knochen zugewandt ist, zumindest in Abschnitten eine offenzellige oder offenporige Oberflächenstruktur (7) besitzt, sowie dadurch, dass mindestens zwei Flügel (8) radial von dem Stiel (4) aus abzweigen, die bis zur Seite (6) des den Knochen zugewandten Kragens reichen, und die Flügel (8) zumindest in Abschnitten eine offenzellige oder offenporige Oberflächenstruktur (7) besitzen.

2. Schulter-Endoprothese nach einem der Ansprüche 1, **dadurch gekennzeichnet, dass** mindestens zwei Flügel (8) medial ausgerichtet sind.

3. Schulter-Endoprothese nach Anspruch 1, **dadurch gekennzeichnet, dass** sich der Gelenkkopf (2) von seiner Rotationsachse (3) aus radial bis zum äußeren Bereich des Kragens (5) erstreckt.

4. Schulter-Endoprothese nach Anspruch 3, **dadurch gekennzeichnet, dass** der äußere Bereich des Kragens (5) axial an den Gelenkkopf (2) angrenzt.

5. Schulter-Endoprothese nach Anspruch 4, **dadurch gekennzeichnet, dass** der äußere Bereich des Kragens (5) zu der Oberfläche des Gelenkkopfes (2) passt, so dass der Gelenkkopf (2) und der Kragen (5) eine kugelförmige Kammer bilden.

6. Schulter-Endoprothese nach Anspruch 1, **dadurch gekennzeichnet, dass** der Gelenkkopf (2) axial über den äußeren Bereich des Kragens (5) hinausragt.

7. Schulter-Endoprothese nach einem der vorhergehenden Ansprüche, **dadurch gekennzeichnet, dass** der Gelenkkopf (2) abnehmbar auf dem Kragen (5) befestigt ist.

8. Schulter-Endoprothese nach einem der vorhergehenden Ansprüche, **gekennzeichnet durch** eine konisch verlaufende Steckverbindung (10, 11) zwischen dem Gelenkkopf und dem Kragen (5).

9. Schulter-Endoprothese nach Anspruch 8, **dadurch gekennzeichnet, dass** sich auf der Unterseite des Gelenkkopfes (2) eine konische Aushöhlung (11) und auf dem Kragen (5) ein konisch verlaufender Stecker (10) befinden.

10. Schulter-Endoprothese nach einem der vorhergehenden Ansprüche, **gekennzeichnet durch** ein Kopplungselement (13) zwischen dem Gelenkkopf (2) und dem Kragen (5) mit einem inneren Konus (14) und einem äußeren Konus (15), so dass der innere Konus (14) mit dem konisch verlaufenden Stecker (10) und der äußere Konus (15) mit der konischen Aushöhlung (11) gekoppelt werden können.

11. Schulter-Endoprothese nach einem der vorhergehenden Ansprüche, **dadurch gekennzeichnet, dass** sich die Rotationsachse (3) des Gelenkkopfes (2) in einem spezifischen Winkel zur Seite (6) des Kragens (5), die den Knochen zugewandt ist, befindet.

12. Schulter-Endoprothese nach Anspruch 10 oder 11, **dadurch gekennzeichnet, dass** die Rotationsachsen des inneren Konus (14) und des äußeren Konus (15) des Kopplungselements (13) zueinander einen bestimmten Winkel α bilden.

13. Schulter-Endoprothese nach einem der vorhergehenden Ansprüche, **dadurch gekennzeichnet, dass** auf der Unterseite des Gelenkkopfes (2) eine Aushöhlung vorhanden ist, die zum Kragen (5) passt.

14. Schulter-Endoprothese nach einem der vorhergehenden Ansprüche, **dadurch gekennzeichnet, dass** sich der Stiel (4) zu seinem distalen Ende hin verengt.

## Revendications

1. Endoprothèse d'épaule comportant une parie humérale (1) comprenant une tige (4) qui peut être ancrée dans l'humérus, une collerette (5) à l'extrémité proximale de la tige (4), et une tête d'articulation (2) en forme de segment de sphère sur la collerette (5), la collerette (5) étant conçue comme un disque globalement circulaire et la tête d'articulation (2) s'étendant dans le sens radial à partir de son axe de rotation (3) au moins jusqu'à la périphérie de la collerette (5),
**caractérisée en ce**
**que** la collerette (5) a, sur son côté (6) faisant face aux os, au moins sur certaines sections, une structure de surface à cellules ouvertes ou à pores ouverts (7), qu'au moins deux ailes (8) qui s'étendent jusqu'au côté (6) de la collerette (5) faisant face à l'os partent de la tige (4) dans le sens radial et que les ailes (8) ont, au moins sur certaines sections, une structure de surface à cellules ouvertes ou à pores ouverts (7).

2. Endoprothèse d'épaule selon une des revendications 1, **caractérisée en ce qu'**au moins deux ailes (8) sont orientées dans le sens médian.

3. Endoprothèse d'épaule selon la revendication 1, **caractérisée en ce que** la tête d'articulation (2) s'étend dans le sens radial à partir de son axe de rotation (3) jusqu'à la périphérie de la collerette (5).

4. Endoprothèse d'épaule selon la revendication 3, **caractérisée en ce que** la périphérie de la collerette (5) est adjacente dans le sens axial à la tête d'articulation (2).

5. Endoprothèse d'épaule selon la revendication 4, **caractérisée en ce que** la périphérie de la collerette (5) est adaptée à la surface de la tête d'articulation (2), de sorte que la tête d'articulation (2) et la collerette (5) forment une voûte sphérique.

6. Endoprothèse d'épaule selon la revendication 1, **caractérisée en ce que** la tête d'articulation (2) est en projection axiale au delà de la périphérie de la collerette (5).

7. Endoprothèse d'épaule selon une des revendications précédentes, **caractérisée en ce que** la tête d'articulation (2) est fixée de manière amovible à la collerette (5).

8. Endoprothèse d'épaule selon une des revendications précédentes, **caractérisée par** une connexion par prise conique (10, 11) entre la tête d'articulation et la collerette (5).

9. Endoprothèse d'épaule selon la revendication 8, **caractérisée en ce qu'**il y a une cavité conique (11) à la partie inférieure de la tête d'articulation (2) et une prise conique (10) sur la collerette (5).

10. Endoprothèse d'épaule selon une des revendications précédentes, **caractérisée par** un élément d'accouplement (13) entre la tête d'articulation (2) et la collerette (5), comportant un intérieur de cône (14) et un extérieur de cône (15) tels que l'intérieur du cône (14) puisse être couplé à la prise conique (10) et que l'extérieur du cône (15) puisse être couplé à la cavité conique (11).

11. Endoprothèse d'épaule selon une des revendications précédentes, **caractérisée en ce que** l'axe de rotation (3) de la tête d'articulation (2) forme un angle spécifié avec le côté (6) faisant face à l'os de la collerette (5).

12. Endoprothèse d'épaule selon la revendication 10 ou 11, **caractérisée en ce que** les axes de rotation de l'intérieur du cône (14) et de l'extérieur du cône (15) de l'élément d'accouplement (13) forment l'un avec l'autre un angle spécifié á.

13. Endoprothèse d'épaule selon une des revendications précédentes, **caractérisée en ce qu'**une cavité adaptée à la collerette (5) se trouve sur la partie inférieure de la tête d'articulation (2).

14. Endoprothèse d'épaule selon une des revendications précédentes, **caractérisée en ce que** la tige (4) est effilée en direction de son extrémité distale.
